(19) **Europäisches Patentamt**
**European Patent Office**
**Office européen des brevets**

(11) **EP 2 178 448 B1**

(12) **EUROPEAN PATENT SPECIFICATION**

(45) Date of publication and mention
of the grant of the patent:
**10.10.2018 Bulletin 2018/41**

(51) Int Cl.:
***A61B 17/32*** ^(2006.01)

(21) Application number: **08782303.5**

(22) Date of filing: **24.07.2008**

(86) International application number:
**PCT/US2008/070983**

(87) International publication number:
**WO 2009/018075 (05.02.2009 Gazette 2009/06)**

(54) **ULTRASONIC SURGICAL INSTRUMENTS**

ULTRASCHALL-OPERATIONSINSTRUMENTE

INSTRUMENTS CHIRURGICAUX À ULTRASONS

(84) Designated Contracting States:
**AT BE BG CH CY CZ DE DK EE ES FI FR GB GR
HR HU IE IS IT LI LT LU LV MC MT NL NO PL PT
RO SE SI SK TR**

(30) Priority: **27.07.2007 US 881636**
**27.07.2007 US 881645**

(43) Date of publication of application:
**28.04.2010 Bulletin 2010/17**

(73) Proprietor: **Ethicon LLC**
**00969 Guaynabo (PR)**

(72) Inventor: **STULEN, Foster B.**
**Mason, Ohio 45040 (US)**

(74) Representative: **Tunstall, Christopher Stephen et
al**
**Carpmaels & Ransford LLP**
**One Southampton Row**
**London WC1B 5HA (GB)**

(56) References cited:
**EP-A1- 0 238 667        EP-A1- 1 138 264
WO-A1-01/24713        WO-A2-2006/101661
US-A- 3 956 826        US-A- 5 897 569
US-A- 5 897 569        US-A1- 2001 025 184
US-A1- 2002 156 493        US-A1- 2003 204 199
US-B1- 6 425 906        US-E- R E25 033**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**BACKGROUND**

The subject application is related a co-pending and commonly-owned application filed on even date herewith, the application being respectively entitled Ultrasonic Surgical Instruments to Foster B. Stulen (Attorney Docket No. END6122USNP/070125, US Application No. 11/881,643).

Ultrasonic instruments, including both hollow core and solid core instruments, are used for the safe and effective treatment of many medical conditions. Ultrasonic instruments, and particularly solid core ultrasonic instruments, are advantageous because they may be used to cut and/or coagulate tissue using energy in the form of mechanical vibrations transmitted to a surgical end effector at ultrasonic frequencies. Ultrasonic vibrations, when transmitted to organic tissue at suitable energy levels and using a suitable end effector, may be used to cut, dissect, or coagulate tissue or elevate or separate muscle tissue off bone. Ultrasonic instruments utilizing solid core technology are particularly advantageous because of the amount of ultrasonic energy that may be transmitted from the ultrasonic transducer, through an ultrasonic transmission waveguide, to the surgical end effector. Such instruments may be used for open procedures or minimally invasive procedures, such as endoscopic or laparoscopic procedures, wherein the end effector is passed through a trocar to reach the surgical site.

Activating or exciting the single or multiple element end effector (e.g., cutting blade, ball coagulator) of such instruments at ultrasonic frequencies induces longitudinal, transverse, or torsional vibratory movement that generates localized heat within adjacent tissue, facilitating both cutting and coagulating. Because of the nature of ultrasonic instruments, a particular ultrasonically actuated end effector may be designed to perform numerous functions, including, for example, cutting and coagulating.

Ultrasonic vibration is induced in the surgical end effector by electrically exciting a transducer, for example. The transducer may be constructed of one or more piezoelectric or magnetostrictive elements in the instrument hand piece. Vibrations generated by the transducer section are transmitted to the surgical end effector via an ultrasonic waveguide extending from the transducer section to the surgical end effector. The waveguides and end effectors are most preferably designed to resonate at the same frequency as the transducer. When an end effector is attached to a transducer the overall system frequency may be the same frequency as the transducer itself.

The transducer and the end effector may be designed to resonate at two different frequencies and when joined or coupled may resonate at a third frequency. The zero-to-peak amplitude of the longitudinal ultrasonic vibration at the tip, d, of the end effector behaves as a simple sinusoid at the resonant frequency as given by:

$$d = A \sin(\omega t)$$

where:

$\omega$ = the radian frequency which equals $2\pi$ times the cyclic frequency, f; and
A = the zero-to-peak amplitude.

The longitudinal excursion is defined as the peak-to-peak (p-t-p) amplitude, which is just twice the amplitude of the sine wave or 2A.

Solid core ultrasonic surgical instruments may be divided into two types, single element end effector devices and multiple-element end effectors. Single element end effector devices include instruments such as scalpels (e.g., blades, sharp hook blades, dissecting hook blades, curved blades) and ball coagulators. Single-element end effector instruments have limited ability to apply blade-to-tissue pressure when the tissue is soft and loosely supported. Substantial pressure may be necessary to effectively couple ultrasonic energy to the tissue. The inability of a single-element end effector to grasp the tissue results in a further inability to fully coapt tissue surfaces while applying ultrasonic energy, leading to less-than-desired hemostasis and tissue joining. The use of multiple-element end effectors such as clamping coagulators includes a mechanism to press tissue against an ultrasonic blade that can overcome these deficiencies.

Ultrasonic clamp coagulators or clamped coagulating shears provide an improved ultrasonic surgical instrument for cutting/coagulating tissue, particularly loose and unsupported tissue, wherein the ultrasonic blade is employed in conjunction with a clamp for applying a compressive or biasing force to the tissue, whereby faster coagulation and cutting of the tissue.

As the distal end of the end effector, or more particularly, the blade, cuts through or coagulates tissue it comes into contact with fluid (e.g., blood, tissue particles). When the distal end of the blade contacts this fluid, a fine mist in the form of a diverging plume of fluid particles may emanate from the distal end of the blade. This plume of mist may limit visibility at the surgical site. It would be desirable to provide an ultrasonic instrument which reduces the plume of mist emanating from the distal end of the end effector.

US5897569A relates to a generator of ultrasonic signals for a transducer which incorporates at least a phase lock loop feedback system for purposes of maintaining the output frequency between first and second voltage values and also incorporates supervisory control circuitry for monitoring whether or not the generator and the transducer continue to operate in a resonant condition.

USRE25033E relates to the art of ultrasonic machining of materials that is to cutting, grinding, boring, or otherwise altering the configuration of materials including relatively hard and brittle materials, by means of a tool vibrated at sonic or ultrasonic frequencies. The disclosure relates to improved apparatus for carrying out the described process, novel acoustical impedance transformers and tools so correlated to each other as to insure optimum performance of specific machining operations.

US6425906B1 relates to a surgical tool for cutting and/or coagulating tissue with a piezo-electric driver to generate ultrasonic energy including torsional mode vibrations. A waveguide is operatively connected at a proximal end to the driver and extends a distance of is the wavelength of ultrasonic vibration in the material of the work horn or waveguide divided by two. A distal end of the waveguide is provided with a cutting and/or coagulating tool.

EP0238667A1 relates to a surgical instrument for cutting and separating biological tissue by employing ultrasonic vibration, which is connected to a source of ultrasonic vibration and performs mechanical vibration at the frequency of the ultrasonic waves, and includes a spoon-shaped working portion which is provided with at least one of a blade-shaped portion forming a predetermined angle with respect to the direction of mechanical vibration at the frequency of the ultrasonic waves and a blade-shaped portion substantially parallel with the direction of the mechanical vibration, the spoon-shaped working portion being adapted to be brought into contact with the biological tissue, and a liquid passage passing through the interior of the tool, the liquid passage having an opening which opens into the working portion.

## SUMMARY

The present invention is defined by claim 1. Further details of specific embodiments are provided by the dependent claims. Therefore, throughout the description, the term "embodiment" only refers to the present invention when the described embodiment falls within the scope of the claims.

In one general aspect, the various embodiments are directed to a surgical instrument with mist reducing features. The surgical instrument may comprise a transducer configured to produce vibrations at a predetermined frequency. An ultrasonic blade extends along a longitudinal axis and is coupled to the transducer. The ultrasonic blade comprises a body having a proximal end and a distal end. The distal end is movable relative to a longitudinal axis by the vibrations produced by the transducer. The body comprises a treatment region that extends from the proximal end to the distal end. At least a portion of the body comprises at least one layer of a first material to globalize fluid particles in contact therewith.

The body may comprise elements which may lead to the reduction of mist emanating from a distal end of the blade. The body comprises a tapered concave surface which extends inwardly into the distal end of the blade. The tapered concave surface may reduce misting by causing fluid particles to converge once leaving the distal end of the blade as opposed to diverging into a plume. The tapered concave surface may define a variety of symmetrical and asymmetrical shapes including conical, frusto-conical and a partial spheroid. The tapered concave surface may be formed on the distal end of symmetrical as well as asymmetrical ultrasonically actuatable blades. In other embodiments, the body may also comprise additional elements which may lead to the reduction of mist. In one embodiment, the body may comprise at least one layer of a first material which comprises a material suitable to carry an electrical charge. In another embodiment, the body may comprise a longitudinally extending bore formed within the blade.

## FIGURES

The features of the various embodiments are set forth with particularity in the appended claims. The various embodiments, however, both as to organization and methods of operation, may best be understood by reference to the following description, taken in conjunction with the accompanying drawings as follows.

FIG. 1A illustrates one embodiment of an ultrasonic system comprising a single element end effector.
FIG. 1B illustrates one embodiment of an ultrasonic system comprising a multi-element end effector.
FIG. 2 illustrates one embodiment of a connection union/joint for an ultrasonic instrument.
FIG. 3A illustrates an exploded perspective view of one embodiment of a single element end effector ultrasonic surgical instrument that may be coupled to the ultrasonic system illustrated in FIG. 1A.
FIG. 3B illustrates one embodiment of a clamp coagulator comprising a multi-element end effector as shown in FIG. 1B.
FIG. 3C illustrates a perspective view of the multi-element end effector as shown in FIGS. 1B and 3B.
FIGS. 4-6 illustrate one embodiment of an ultrasonic blade, where:

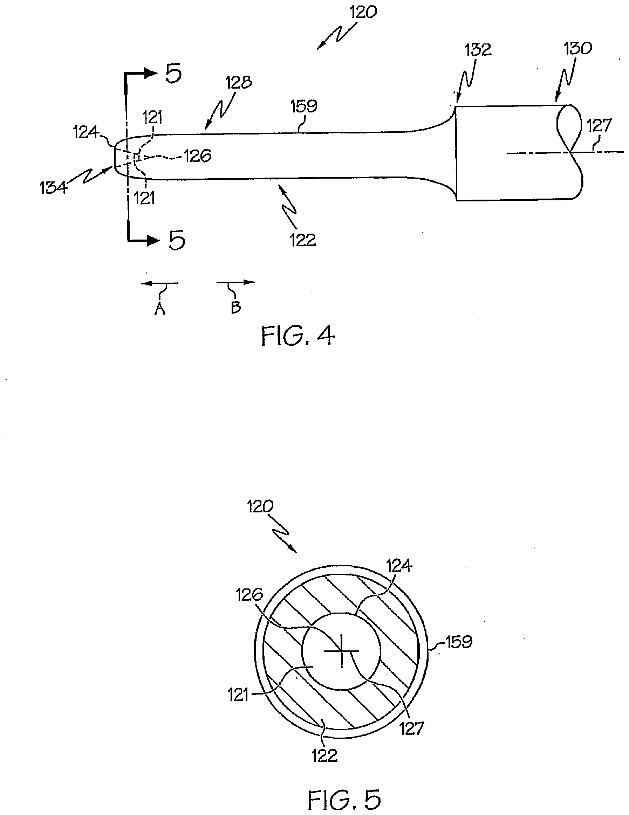

FIG. 4 is a side view of one embodiment of an ultrasonic blade;
FIG. 5 is a cross-sectional view of the ultrasonic blade taken along line 5-5 in FIG. 4; and

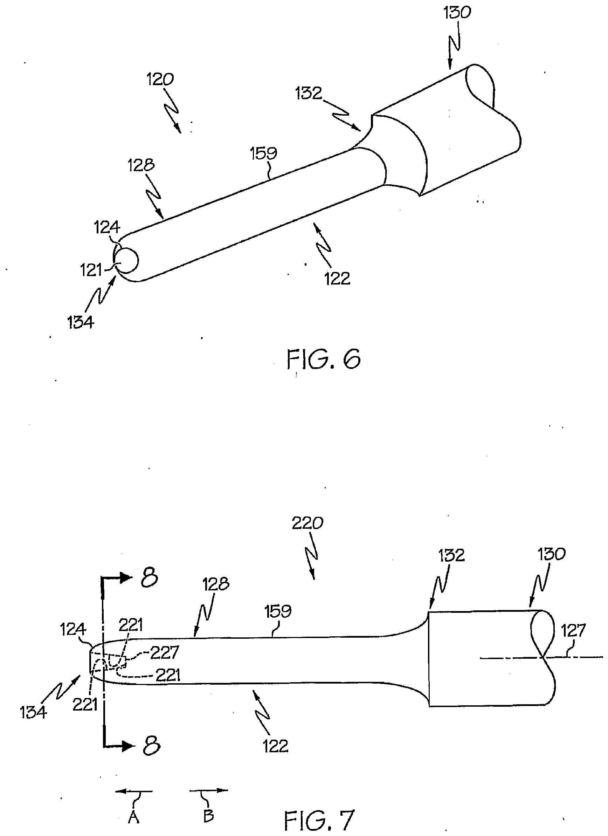

FIG. 6 is a perspective view of the ultrasonic blade shown in Fig. 4.
FIGS. 7-9 illustrate various embodiments of the ultrasonic blade, where:
FIG. 7 is a side view of one embodiment of an ultrasonic blade;

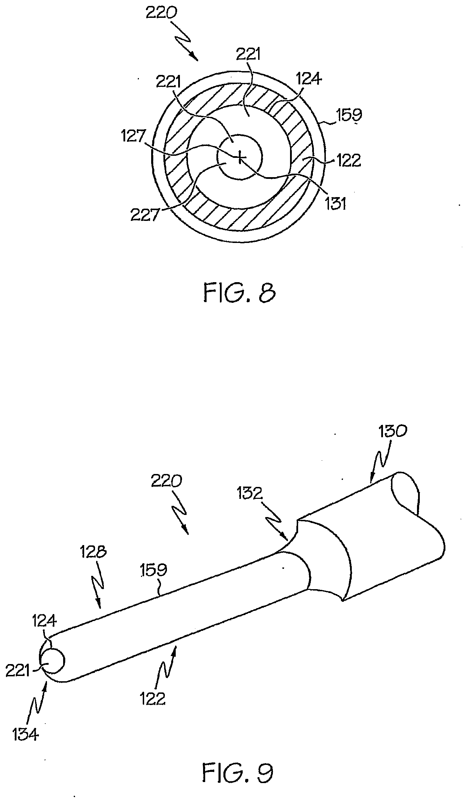

FIG. 8 is a cross-sectional view of the ultrasonic blade taken along line 8-8 in FIG. 7; and
FIG. 9 is a perspective view of the ultrasonic blade shown in Fig. 7.

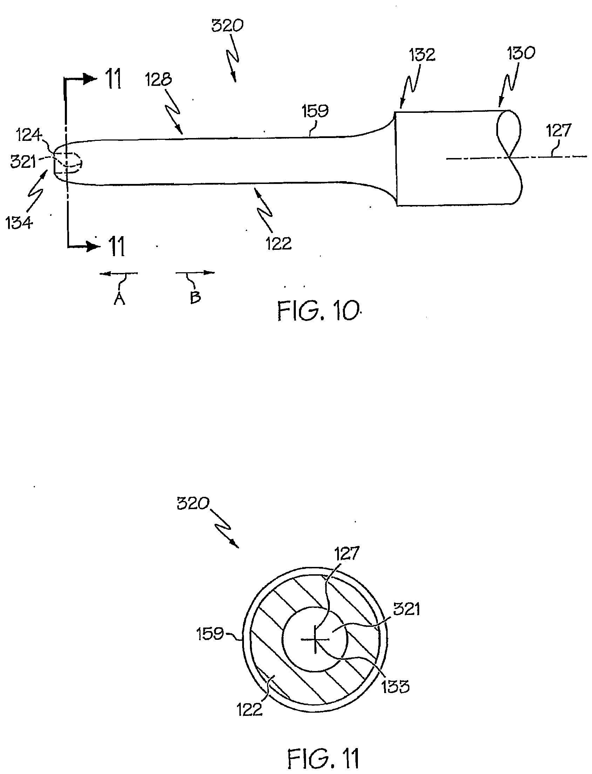

FIGS. 10-12 illustrate one embodiment of the ultrasonic blade, where:

**FIG. 10** is a side view of one embodiment of an ultrasonic blade;

**FIG. 11** is a cross-sectional view of the ultrasonic blade taken along line 11-11 in FIG. 10; and

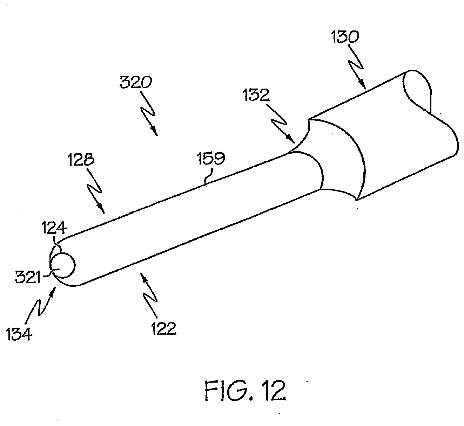

**FIG. 12** is a perspective view of the ultrasonic blade shown in Fig. 10.

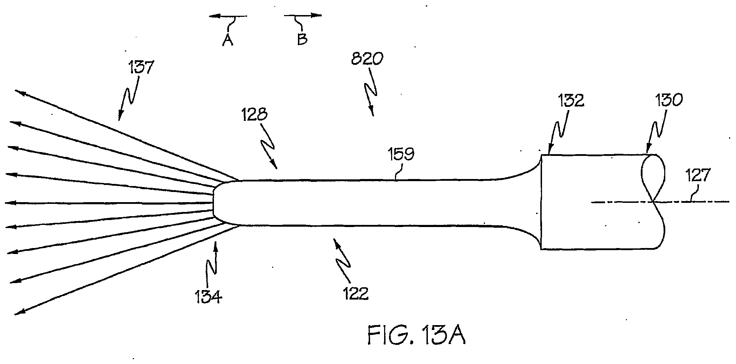

**FIGS. 13A-B** illustrate various embodiments of an ultrasonic blade, where:

**FIG. 13A** is a side view of an ultrasonic blade with a convex blade tip depicting a divergent plume mist; and

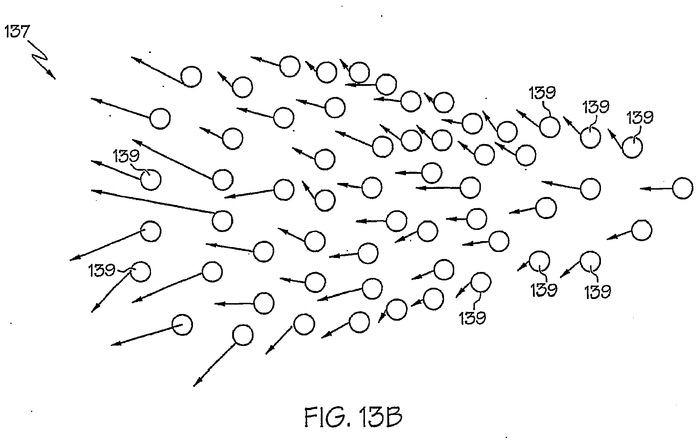

**FIG. 13B** is a detail view of the divergent jet of fluid mist.

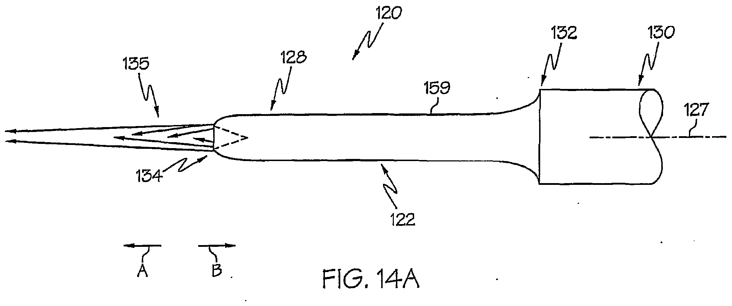

**FIGS. 14A-B** illustrate various embodiments of an ultrasonic blade, where:

**FIG. 14A** is a side view of an ultrasonic blade with a tapered concave surface formed at a distal end of the blade depicting a convergence of the fluid leaving the blade tip; and

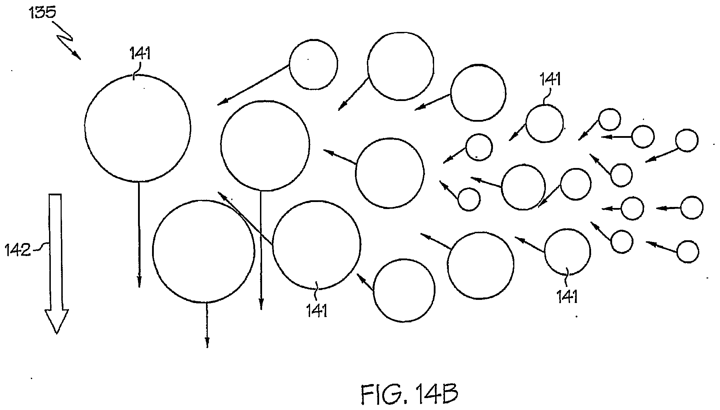

**FIG. 14B** is a detail view of the convergent jet of fluid mist.

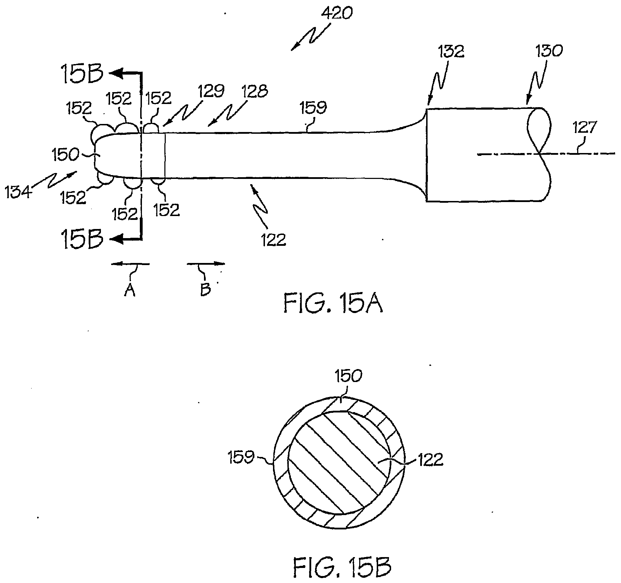

**FIGS. 15A-D** illustrate various embodiments of an ultrasonic blade, where:

**FIG. 15A** is a side view of an ultrasonic blade with at least a portion of the ultrasonic blade coated with at least one layer of a material which may allow the fluid to form globules on the surface of the material; and

**FIG. 15B** is cross-sectional view of the ultrasonic blade taken along line B-B in FIG. 15A.

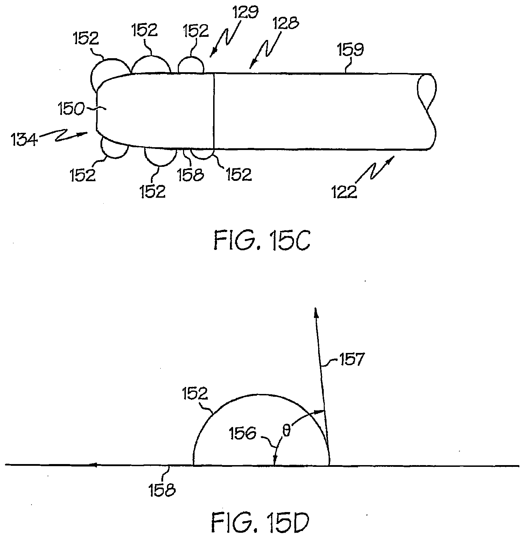

**FIG. 15C** is a detailed view of the ultrasonic blade of FIG. 15A.

**FIG. 15D** illustrates a contact angle between a droplet and the surface of the ultrasonic blade of FIG. 15A.

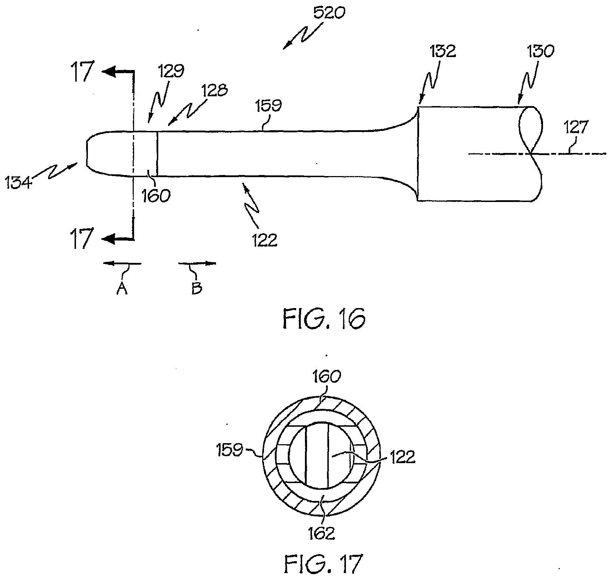

**FIGS. 16-17** illustrate various embodiments of an ultrasonic blade, where:

**FIG. 16** is a side view of an ultrasonic blade with portions of the blade coated with more than one material to provide an electric charge to the blade tip; and

**FIG. 17** is cross-sectional view of the ultrasonic blade taken along line 17-17 in FIG. 16.

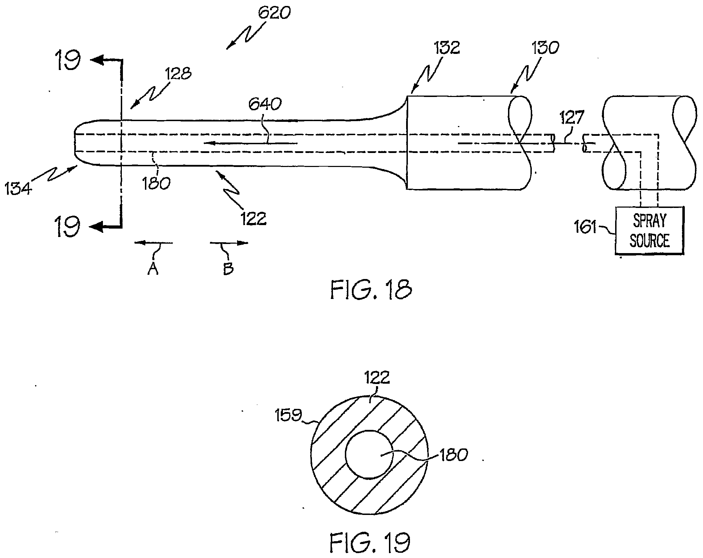

**FIGS. 18-19** illustrate various embodiments of an ultrasonic blade, where:

**FIG. 18** is a side view of an ultrasonic blade with a longitudinally extending bore; and

**FIG. 19** is cross-sectional view of the ultrasonic blade taken along line 19-19 in FIG. 18.

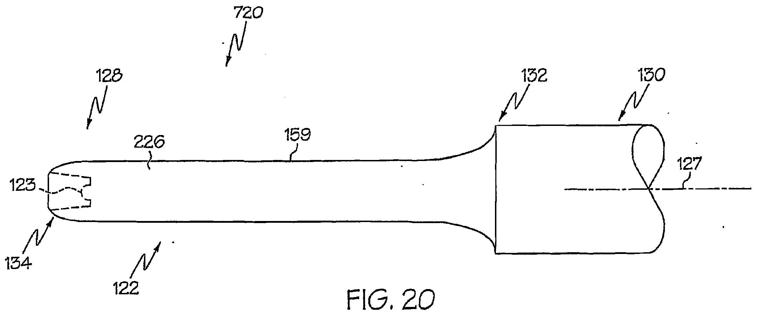

**FIG. 20** is a side view of an ultrasonic blade with a convex portion within a tapered concave surface thereof.

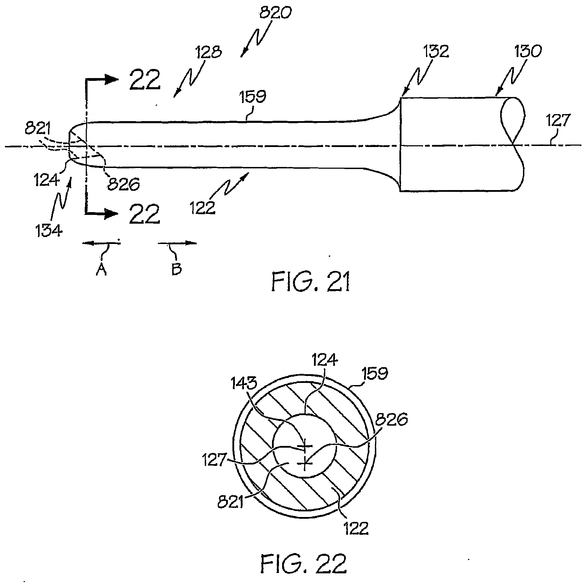

**FIG. 21-22** illustrate various embodiments of an ultrasonic blade, where:

**FIG. 21** is a side view of an ultrasonic blade with a tapered concave surface extending into the blade body asymmetrically.

**FIG. 22** is a cross-sectional view of the ultrasonic blade taken along line 22-22 in FIG. 21.

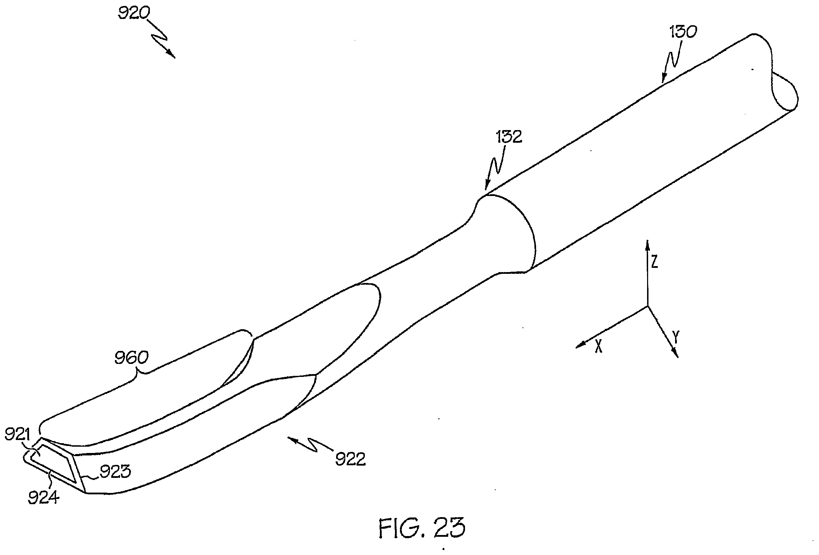

**FIG. 23** is a perspective view of an asymmetric ultrasonic blade comprising a tapered concave surface extending inwardly into the blade body.

## DESCRIPTION

Before explaining the various embodiments in detail, it should be noted that the embodiments are not limited in its application or use to the details of construction and arrangement of parts illustrated in the accompanying drawings and description. The illustrative embodiments may be implemented or incorporated in other embodiments, variations and modifications, and may be practiced or carried out in various ways. For example, the surgical instruments and blade configurations disclosed below are illustrative only. Furthermore, unless otherwise indicated, the terms and expressions employed herein have been chosen for the purpose of describing the illustrative embodiments for the convenience of the reader.

The various embodiments relate, in general, to ultrasonic blades for use in surgical instruments and, more particularly, to ultrasonic blades comprising mist reducing features as described herein. The various embodiments relate, in general, to ultrasonic blades and instruments to improve visibility of the surgical site during surgery by reducing the mist plume created by fluid particles colliding with a distal end of an activated ultrasonic blade. Visibility of the surgical site may be improved through the mist reducing features of the ultrasonic blades which may comprise a tapered concave surface formed at the distal end of the blade, a tip coating, a lumen fluidically coupled to a spraying mechanism, a material to hold an electric charge, or any combination thereof. The term "tapered concave surface" is defined as a concave surface formed at a distal end of the blade that is tapered inwardly from its distal end to its proximal end in the direction indicated by arrow B, various embodiments of which are shown in **FIGS. 4-23.** A variety of different blade configurations are disclosed which may be useful for both open and laparoscopic applications.

Examples of ultrasonic surgical instruments are disclosed in U.S. Pat. Nos. 5,322,055 and 5,954,736 and in combination with ultrasonic blades and surgical instruments disclosed in U.S. Pat. Nos. 6,309,400 B2, 6,278,218 B1, 6,283,981 B1, and 6,325,811 B1, for example. These references disclose ultrasonic surgical instruments and blade configurations where a longitudinal mode of the blade is excited. Because of asymmetry or asymmetries, ultrasonic blades also may exhibit transverse and/or torsional motion where the characteristic "wavelength" of this non-longitudinal motion is generally less than that of the general longitudinal motion of the blade and its extender portion. Therefore, the wave shape of the non-longitudinal motion will present nodal positions of transverse/torsional motion along the tissue effector while the net motion of the active blade along its tissue effector is non-zero (i.e., will have at least longitudinal motion along the length extending from its distal end, an antinode of longitudinal motion, to the first nodal position of longitudinal motion that is proximal to the tissue effector portion).

Certain embodiments will now be described to provide an overall understanding of the principles of the structure, function, manufacture, and use of the devices and methods disclosed herein. One or more examples of these embodiments are illustrated in the accompanying drawings. Those of ordinary skill in the art will understand that the devices and methods specifically described herein and illustrated in the accompanying drawings are non-limiting embodiments and that the scope of the various embodiments is defined solely by the claims. The features illustrated or described in connection with one embodiment may be combined with the features of other embodiments.

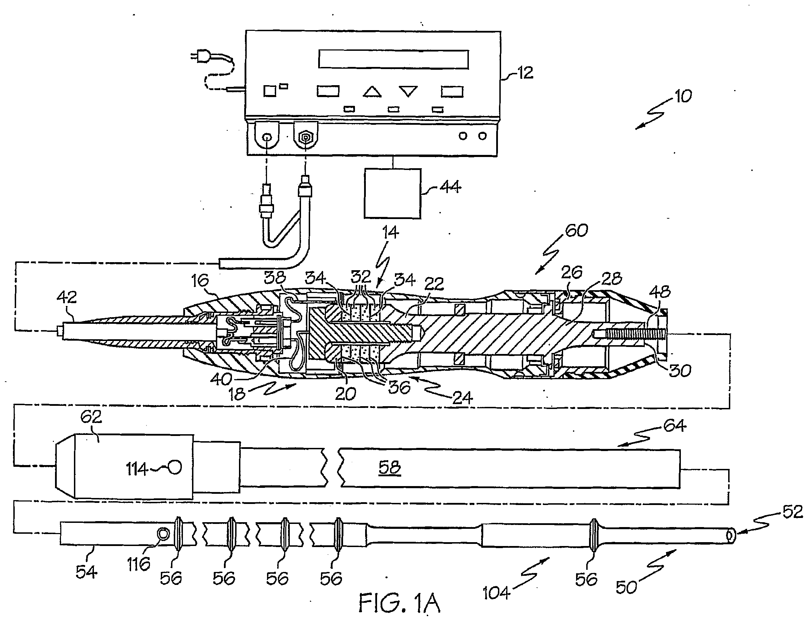

FIG. 1A illustrates one embodiment of an ultrasonic system 10 comprising a single element end effector. One embodiment of the ultrasonic system 10 comprises an ultrasonic signal generator 12 coupled to an ultrasonic transducer 14, a hand piece assembly 60 comprising a hand piece housing 16, and an ultrasonically actuatable single element end effector or ultrasonically actuatable blade 50. The ultrasonic transducer 14, which is known as a "Langevin stack", generally includes a transduction portion 18, a first resonator portion or end-bell 20, and a second resonator portion or fore-bell 22, and ancillary components. The total construction of these components is a resonator. The ultrasonic transducer 14 is preferably an integral number of one-half system wavelengths ($n\lambda/2$; where "n" is any positive integer; e.g., n = 1, 2, 3...) in length as will be described in more detail later. An acoustic assembly 24 includes the ultrasonic transducer 14, a nose cone 26, a velocity transformer 28, and a surface 30.

It will be appreciated that the terms "proximal" and "distal" are used herein with reference to a clinician gripping the hand piece assembly 60. Thus, the blade 50 is distal with respect to the more proximal hand piece assembly 60. It will be further appreciated that, for convenience and clarity, spatial terms such as "top" and "bottom" also are used herein with respect to the clinician gripping the hand piece assembly 60. However, surgical instruments are used in many orientations and positions, and these terms are not intended to be limiting and absolute.

The distal end of the end-bell 20 is connected to the proximal end of the transduction portion 18, and the proximal end of the fore-bell 22 is connected to the distal end of the transduction portion 18. The fore-bell 22 and the end-bell 20 have a length determined by a number of variables, including the thickness of the transduction portion 18, the density and modulus of elasticity of the material used to manufacture the end-bell 20 and the fore-bell 22, and the resonant frequency of the ultrasonic transducer 14. The fore-bell 22 may be tapered inwardly from its proximal end to its distal end to amplify the ultrasonic vibration amplitude as the velocity transformer 28, or alternately may have no amplification. A suitable vibrational frequency range may be about 20Hz to 120kHz and a well-suited vibrational frequency range may be about 30-100kHz. A suitable operational vibrational frequency may be approximately 55.5kHz, for example.

Piezoelectric elements 32 may be fabricated from any suitable material, such as, for example, lead zirconate-titanate, lead meta-niobate, lead titanate, barium titanate, or other piezoelectric ceramic material. Each of positive electrodes 34, negative electrodes 36, and the piezoelectric elements 32 has a bore extending through the center. The positive and negative electrodes 34 and 36 are electrically coupled to wires 38 and 40, respectively. The wires 38 and 40 are encased within a cable 42 and electrically connectable to the ultrasonic signal generator 12 of the ultrasonic system 10.

The ultrasonic transducer 14 of the acoustic assembly 24 converts the electrical signal from the ultrasonic signal generator 12 into mechanical energy that results in primarily a standing acoustic wave of longitudinal vibratory motion of the ultrasonic transducer 14 and the end effector 50 at ultrasonic frequencies. In another embodiment, the vibratory motion of the ultrasonic transducer may act in a different direction. For example, the vibratory motion may comprise a local longitudinal component of a more complicated motion of the tip of the ultrasonic system 10. A suitable generator is available as model number GEN04, from Ethicon Endo-Surgery, Inc., Cincinnati, Ohio. When the acoustic assembly 24 is energized, a vibratory motion standing wave is generated through the acoustic assembly 24. The ultrasonic system 10 is designed to operate at a resonance such that an acoustic standing wave pattern of predetermined amplitude is produced. The amplitude of the vibratory motion at any point along the acoustic assembly 24 depends upon the location along the acoustic assembly 24 at which the vibratory motion is measured. A minimum or zero crossing in the vibratory motion standing wave is generally referred to as a node (i.e., where motion is minimal), and a local absolute value maximum or peak in the standing wave is generally referred to as an anti-node (i.e., where local motion is maximal). The distance between an anti-node and its nearest node is one-quarter wavelength ($\lambda/4$).

The wires 38 and 40 transmit an electrical signal from the ultrasonic signal generator 12 to the positive electrodes 34 and the negative electrodes 36. The piezoelectric elements 32 are energized by the electrical signal supplied from the ultrasonic signal generator 12 in response to an actuator 44, such as a foot switch, for example, to produce an acoustic standing wave in the acoustic assembly 24. The electrical signal causes disturbances in the piezoelectric elements 32 in the form of repeated small displacements resulting in large alternating compression and tension forces within the material. The repeated small displacements cause the piezoelectric elements 32 to expand and contract in a continuous manner along the axis of the voltage gradient, producing longitudinal waves of ultrasonic energy. The ultrasonic energy is transmitted through the acoustic assembly 24 to the single element end effector such as the blade 50 via a transmission component or an ultrasonic transmission waveguide 104.

In order for the acoustic assembly 24 to deliver energy to the single element end effector 50, all components of the acoustic assembly 24 must be acoustically coupled to the blade 50. The distal end of the ultrasonic transducer 14 may be acoustically coupled at the surface 30 to the proximal end of the ultrasonic transmission waveguide 104 by a threaded connection such as a stud 48.

The components of the acoustic assembly 24 are preferably acoustically tuned such that the length of any assembly is an integral number of one-half wavelengths ($n\lambda/2$), where the wavelength $\lambda$ is the wavelength of a pre-selected or operating longitudinal vibration drive frequency $f_d$ of the acoustic assembly 24. It is also contemplated that the acoustic assembly 24 may incorporate any suitable arrangement of acoustic elements.

The blade 50 may have a length substantially equal to an integral multiple of one-half system wavelengths ($n\lambda/2$). A distal end 52 of the blade 50 may be disposed near an antinode in order to provide the maximum longitudinal excursion of the distal end. When the transducer assembly is energized, the distal end 52 of the blade 50 may be configured to move in the range of, for example, approximately 10 to 500 microns peak-to-peak, and preferably in the range of about 30 to 150 microns at a predetermined vibrational frequency of 55kHz, for example.

The blade 50 may comprise features to reduce misting. For example, the blade 50 may comprise a tapered concave surface at the distal end 52, a coating formed at the distal end 52, a lumen fluidically coupled to a spraying mechanism, a material to hold an electric charge, or any combination thereof.

The blade 50 may be coupled to the ultrasonic transmission waveguide 104. The blade 50 and the ultrasonic transmission waveguide 104 as illustrated are formed as a single unit construction from a material suitable for transmission of ultrasonic energy. Examples of such materials include Ti6Al4V (an alloy of Titanium including Aluminum and Vanadium), Aluminum, Stainless Steel, or other suitable materials. Alternately, the blade 50 may be separable (and of differing composition) from the ultrasonic transmission waveguide 104, and coupled by, for example, a stud, weld, glue, quick connect, or other suitable known methods. The length of the ultrasonic transmission waveguide 104 may be substantially equal to an integral number of one-half wavelengths ($n\lambda/2$), for example. The ultrasonic transmission waveguide 104 is fabricated from a solid core shaft constructed out of material suitable to propagate ultrasonic energy efficiently, such as the titanium alloy discussed above (i.e., Ti6Al4V) or any suitable aluminum alloy, or other alloys, for example.

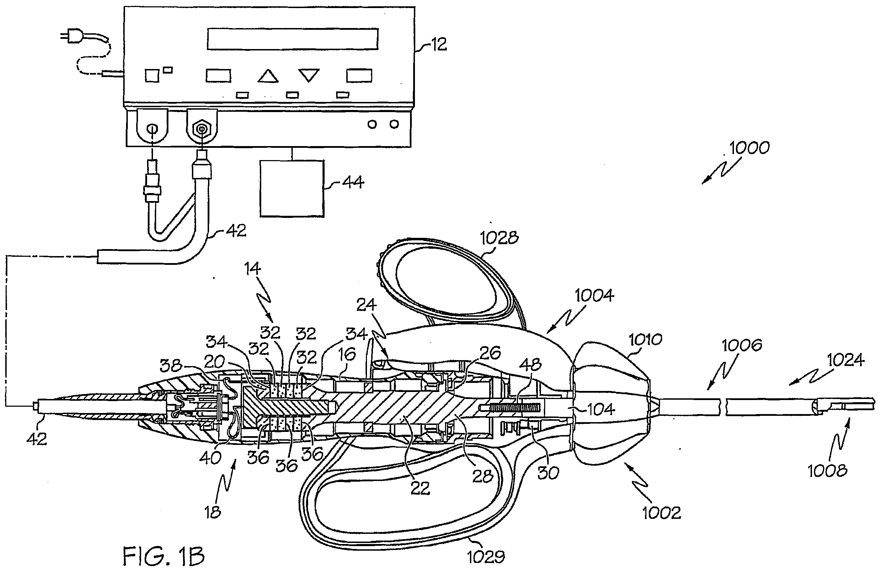

The ultrasonic transmission waveguide 104 comprises a longitudinally projecting attachment post 54 at a proximal end to couple to the surface 30 of the ultrasonic transmission waveguide 104 by a threaded connection such as the stud 48. In the embodiment illustrated in FIG. 1, the ultrasonic transmission waveguide 104 includes a plurality of stabilizing silicone rings or compliant supports 56 positioned at a plurality of nodes. The silicone rings 56 dampen undesirable vibration and isolate the ultrasonic energy from an outer sheath 58 assuring the flow of ultrasonic energy in a longitudinal direction to the distal end 52 of the blade 50 with maximum efficiency.

As shown in FIG. 1, the outer sheath 58 protects a user of the ultrasonic surgical instrument 10, 100 and a patient from the ultrasonic vibrations of the ultrasonic transmission waveguide 104. The sheath 58 generally includes a hub 62 and an elongated tubular member 64. The tubular member 64 is attached to the hub 62 and has an opening extending longitudinally therethrough. The sheath 58 is threaded onto the distal end of the housing 16. The ultrasonic transmission waveguide 104 extends through the opening of the tubular member 64 and the silicone rings 56 isolate the ultrasonic transmission waveguide 104 from the outer sheath 58. The outer sheath 58 may be attached to the waveguide 104 with an isolator pin 112. The hole in the waveguide 104 may occur nominally at a displacement. The waveguide 104 may screw or snap onto the hand piece assembly 60 by the stud 48. The flat portions on the hub 62 may allow the assembly to be torqued to a required level.

The hub 62 of the sheath 58 is preferably constructed from plastic and the tubular member 64 is fabricated from stainless steel. Alternatively, the ultrasonic transmission waveguide 104 may comprise polymeric material surrounding it to isolate it from outside contact.

The distal end of the ultrasonic transmission waveguide 104 may be coupled to the proximal end of the blade 50 by an internal threaded connection, preferably at or near an antinode. It is contemplated that the blade 50 may be attached to the ultrasonic transmission waveguide 104 by any suitable means, such as a welded joint or the like. Although the blade 50 may be detachable from the ultrasonic transmission waveguide 104, it is also contemplated that the single element end effector (e.g., the blade 50) and the ultrasonic transmission waveguide 104 may be formed as a single unitary piece.

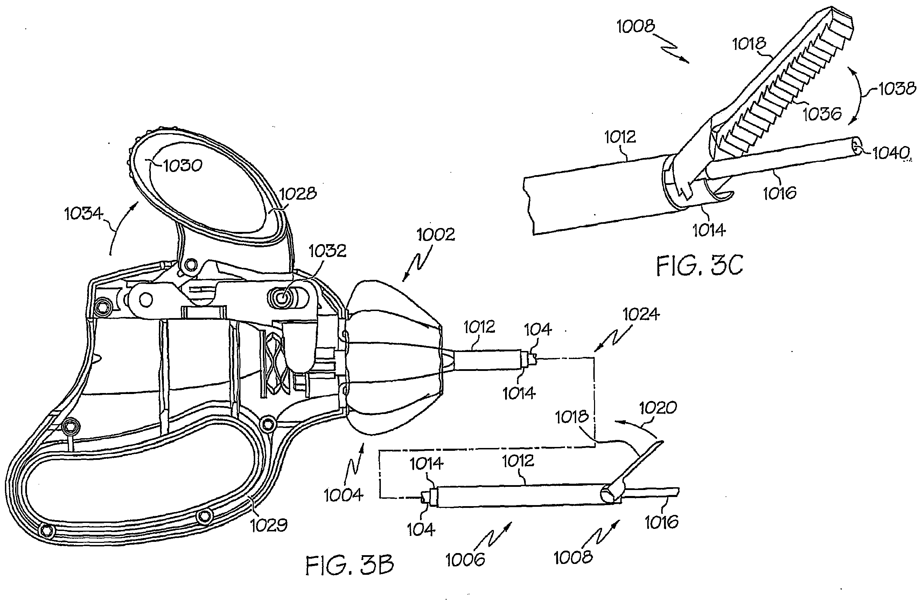

FIG. 1B illustrates one embodiment of an ultrasonic system 1000 comprising a multi-element end effector. One embodiment of the ultrasonic system 1000 comprises the ultrasonic generator 12 coupled to the ultrasonic transducer 14 described with reference to FIG. 1A. The ultrasonic transducer 14 is coupled to clamped coagulating shears 1002 comprising an instrument housing 1004. The acoustic assembly 18 delivers energy to the end effector 1016 (FIG. 3B) of the multi-element end assembly 1008 of the multi-element instrument. In order for the acoustic assembly 18 to deliver energy to the multi-element end effector or multi-element end assembly 1008, all components of the acoustic assembly 18 must be acoustically coupled to the ultrasonically active portions of the clamped coagulating shears 1002. Accordingly, the distal end of the ultrasonic transducer 14 may be acoustically coupled at the surface 30 to the proximal end of the ultrasonic transmission waveguide 104 by the threaded connection stud 48.

As previously discussed with reference to the ultrasonic system 10 shown in FIG. 1A, the components of the acoustic assembly 18 are preferably acoustically tuned such that the length of any assembly is an integral number of one-half wavelengths (nλ/2), where the wavelength λ is the wavelength of a pre-selected or operating longitudinal vibration drive frequency $f_d$ of the acoustic assembly 18. The acoustic assembly 18 may incorporate any suitable arrangement of acoustic elements.

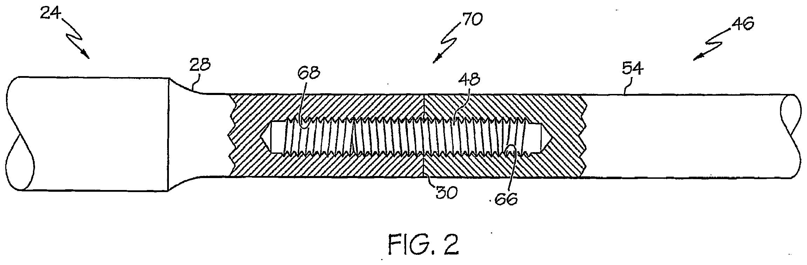

FIG. 2 illustrates one embodiment of a connection union/joint 70 for an ultrasonic instrument. The connection union/joint 70 may be formed between the attachment post 54 of the ultrasonic transmission waveguide 104 and the surface 30 of the velocity transformer 28 at the distal end of the acoustic assembly 24. The proximal end of the attachment post 54 comprises a female threaded substantially cylindrical recess 66 to receive a portion of the threaded stud 48 therein. The distal end of the velocity transformer 28 also may comprise a female threaded substantially cylindrical recess 68 to receive a portion of the threaded stud 40. The recesses 66, 68 are substantially circumferentially and longitudinally aligned. In another embodiment (not shown), the stud is an integral component of the end of the ultrasonic transducer. For example, the treaded stud and the velocity transformer may be of a single unit construction with the stud projecting from a distal surface of the velocity transformer at the distal end of the acoustic assembly. In this embodiment, the stud is not a separate component and does not require a recess in the end of the transducer.

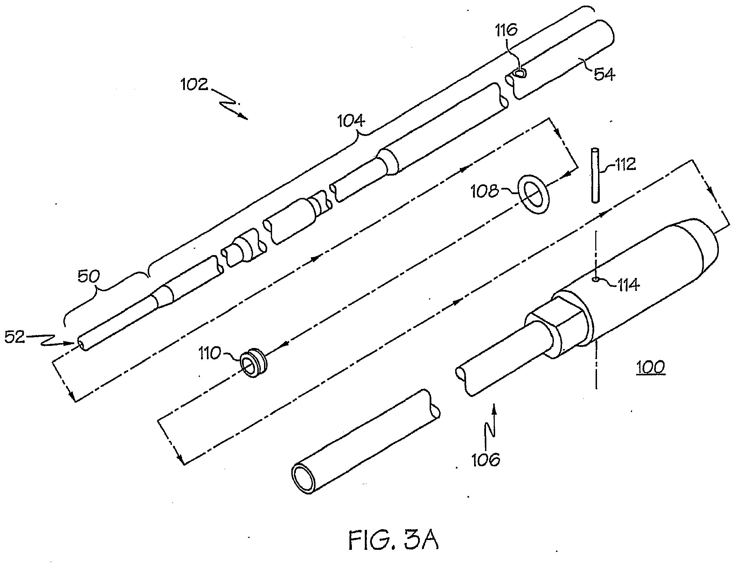

FIG. 3A illustrates an exploded perspective view of one embodiment of a single element end effector ultrasonic surgical instrument 100. The ultrasonic surgical instrument 100 may be employed with the ultrasonic system 10 illustrated in FIG. 1A. However, as described herein, those of ordinary skill in the art will understand that the various embodiments of the ultrasonic surgical instruments disclosed herein as well as any equivalent structures thereof could conceivably be effectively used in connection with other known ultrasonic surgical instruments.

In the embodiment illustrated in FIG. 3A, the elongated transmission component is shown as the ultrasonic waveguide 104 and the end effector is shown as a single element end effector or blade 50 suitable to cut and/or coagulate tissue. The blade 50 may be symmetrical or asymmetrical.

The length of the blade 50 may be substantially equal to an integral multiple of one-half system wavelengths (nλ/2). The distal end 52 of the blade 50 may be disposed near an anti-node in order to provide the maximum longitudinal excursion of the distal end 52. When the transducer assembly is energized, the distal end 52 of the blade 50 may be configured to move in the range of, for example, approximately 10 to 500 microns peak-to-peak, and preferably in the range of about 30 to 150 microns at a predetermined vibrational frequency.

The blade 50 may be coupled to the ultrasonic transmission waveguide 104. The blade 50 and the ultrasonic transmission guide 104 as illustrated are formed as a single unit of construction from a material suitable for transmission of ultrasonic energy such as, for example, Ti6Al4V (an alloy of titanium including aluminum and vanadium), aluminum, stainless steel, other known materials, or combinations thereof. Alternately, the blade 50 may be separable (and of differing composition) from the ultrasonic transmission waveguide 104, and coupled by, for example, a stud, weld, glue, quick connect, or other suitable known methods. The length of the ultrasonic transmission waveguide 104 may be substantially equal to an integral number of one-half system wavelengths (nλ/2), for example. The ultrasonic transmission waveguide 104 also may be preferably fabricated from a solid core shaft constructed out of material that propagates ultrasonic energy efficiently, such as titanium alloy (e.g., Ti6Al4V) or an aluminum alloy, for example. The ultrasonic transmission waveguide 104 also may be fabricated from a hollow core shaft constructed out of similar materials. The ultrasonic transmission waveguide 104 also may be fabricated with a combination solid/hollow core shaft, for example, a solid core shaft with hollow cavities positioned at various locations along the length of the shaft.

In the embodiment illustrated in FIG. 3A, the ultrasonic transmission waveguide 104 is positioned within the outer sheath 58 by a mounting O-ring 108 and a sealing ring 110. In other embodiments, one or more additional dampers or support members (not shown) also may be included along the ultrasonic transmission waveguide 104. The ultrasonic transmission waveguide 104 is affixed to the outer sheath 58 by the mounting pin 112 that passes through mounting holes 114 in the outer sheath 58 and a mounting hole 116 formed in the ultrasonic transmission waveguide 104.

FIG. 3B illustrates one embodiment of the clamped coagulating shears 1002 comprising a multi-element end effector as shown in FIG. 1B. FIG. 3C illustrates a perspective view of the multi-element end effector as shown in FIGS. 1B and 3B. With reference to FIGS. 1B, 3B and 3C, the clamped coagulating shears 1002 may be preferably attached to and removed from the acoustic assembly 18 as a unit. The proximal end of the clamped coagulating shears 1002 preferably acoustically couples to the distal surface 30 of the acoustic assembly 18. The clamped coagulating shears 1002 may be coupled to the acoustic assembly 18 by any suitable means.

The clamped coagulating shears 1002 preferably includes an instrument housing 1004 and an elongated member 1006. The elongated member 1006 may be selectively rotated with respect to the instrument housing 1004. The instrument housing 1004 includes a pivoting handle portion 1028 and a fixed handle portion 1029.

An indexing mechanism (not shown) is disposed within a cavity of the instrument housing 1004. The indexing mechanism is preferably coupled or attached on an inner tube 1014 to translate movement of the pivoting handle portion 1028 to linear motion of the inner tube 1014 to open and close the multi-element end assembly 1008. When the pivoting handle portion 1028 is moved toward the fixed handle portion 1029, the indexing mechanism slide the inner tube 1014 rearward to pivot the multi-element end assembly 1008 into a closed position. The movement of the pivoting handle portion 1028 in the opposite direction slides the indexing mechanism to displace the inner tube 1014 in the opposite direction, i.e., forwardly, and hence pivot the multi-element end assembly 1008 into its open position in the direction indicated by arrow 1020 as shown in FIG. 3B.

The pivoting handle portion 1028 includes a thumb loop 1030. A pivot pin 1032 is disposed through a first hole of the pivoting handle portion 1028 to allow pivoting as shown by arrow 1034 in FIG. 3B. As the thumb loop 1030 of the pivoting handle portion 1028 is moved in the direction of arrow 1034, away from the instrument housing 1004, the inner tube 1014 slides rearward to pivot the multi-element end assembly 1008 into a closed position.

The elongated member 1006 of the clamped coagulating shears 1002 extends from the instrument housing 1004. The elongated member 1006 preferably includes an outer member or outer tube 1012, an inner member or inner tube 1014, and a transmission component or ultrasonic transmission waveguide 104.

The multi-element end effector or multi-element end assembly 1008 includes a clamp arm assembly 1018, a tissue pad 1036, and an ultrasonic blade 1016. The clamp arm assembly 1018 is pivotally mounted about a pivot pin (not shown) to rotate in the direction indicated by arrow 1038. The ultrasonic blade 1016 comprises a tapered concave surface 1040 extending inwardly into the blade body.

The ultrasonic surgical instrument 100 and the clamped coagulating shears 1002 may be sterilized by methods known in the art such as, for example, gamma radiation sterilization, Ethelyne Oxide processes, autoclaving, soaking in sterilization liquid, or other known processes. In the embodiment illustrated in FIGS. 1A and 3A, an ultrasonic transmission assembly 102 of the surgical instrument 100 includes the single element ultrasonically actuated end effector or blade 50 coupled to the ultrasonic transmission waveguide 104. The blade 50 and the ultrasonic transmission waveguide 104 are illustrated as a single unit construction from a material suitable for transmission of ultrasonic energy as previously discussed (e.g., Ti6Al4V, Aluminum, Stainless Steel, or other known materials). Alternately, the blade 50 may be separable (and of differing composition) from the ultrasonic transmission waveguide 104, and coupled by, for example, a stud, weld, glue, quick connect, or other known methods. In the embodiment illustrated in FIGS. 1B and 3B, the ultrasonic transmission assembly 1024 of the clamped coagulating shears 1002 includes the multi-element end assembly 1008 coupled to the ultrasonic transmission waveguide 104. The length of the ultrasonic transmission waveguide 104 may be substantially equal to an integral number of one-half system wavelengths ($n\lambda/2$), for example. The ultrasonic transmission waveguide 104 may be preferably fabricated from a solid core shaft constructed out of material that propagates ultrasonic energy efficiently, such as titanium alloy (i.e., Ti6Al4V) or an aluminum alloy, for example.

FIGS. 4-22 illustrate various embodiments of ultrasonic blades, which may be considered different embodiments of the single element end effector or the blade 50 or the ultrasonic blade 1016 of the multi-element end assembly 1008 and are generally well-suited for cutting, coagulating, and reshaping tissue. In addition, these blades comprise mist reducing features. The ultrasonic blades may be employed in the above-described ultrasonic systems 10, 1000. Those skilled in the art will appreciate that although the various embodiments of the ultrasonic blades 50, 1016 are well-suited for cutting, coagulating, reshaping tissue, and reducing the mist associated with the previously discussed functions, these ultrasonic blades are multifunctional and may be employed in multiple numerous applications.

FIGS. 4-6 illustrate one embodiment of an ultrasonic blade 120. The ultrasonic blade 120 is generally well-suited for cutting, coagulating, and reshaping tissue. The ultrasonic blade 120 may be employed in various other therapeutic procedures. The ultrasonic blade 120 comprises mist reducing features as described herein. FIG. 4 is a side view of one embodiment of the ultrasonic blade 120. FIG. 5 is a cross-sectional view of one embodiment of the ultrasonic blade 120 taken along line 5-5 in FIG. 4. FIG. 6 is a perspective view of one embodiment of the ultrasonic blade in FIG. 4.

In the embodiment illustrated in FIGS. 4-6, the ultrasonic blade 120 comprises a blade body 122 having a proximal end 132 and a distal end 134. As shown in the cross-sectional view of FIG. 5, the body 122 may have a substantially circular cross section. The blade body 122 may extend along a longitudinal central axis 127. The blade body 122 may comprise a tapered concave surface 121 at the distal end 134 of the blade body 122 which may extend inwardly into the blade body 122. This inward extension may occur such that the blade body has an inwardly tapered concave shaped tip as opposed to a conventional convex shaped tip that extends outwardly or a flat faced tip. The blade body 122 may comprise a substantially elongated treatment region 128 and a neck or transition portion 130 that protrudes from the proximal end 132 of the treatment region 128. The neck portion 130 may be configured to attach to the ultrasonic transmission waveguide 104 by a stud, weld, glue, quick connect, or other suitable attachment methods, for example. In various other embodiments, the ultrasonic blade 120 and the ultrasonic transmission waveguide 104 may be formed as a single unitary body. In either configuration, the ultrasonic transmission waveguide 104 may have gain steps to amplify the mechanical vibrations transmitted to the ultrasonic blade 120 as is well known in the art. The ultrasonic blade 120 is adapted to couple to the ultrasonic transmission waveguide 104, which may be employed with the above-described ultrasonic surgical system 10.

In various embodiments, the tapered concave surface 121 may extend inwardly into the blade body 122 from a first edge 124 which may be located at the distal end 134 of the blade body 122. As previously discussed, the surface 121 may be substantially concave and may be tapered inwardly into the blade body 122. In one embodiment, as illustrated in **FIG. 20,** the concave surface 121 may comprise a convex portion 123 or "bump" within the concave surface 121. FIG. 20 is a side view of an ultrasonic blade 720 with the convex portion 123 formed within the concave surface 121. For example, the substantially concave surface may have a convex portion 123 or "bump" extending in a direction different from the inward direction of the extension of the surface 121 (see FIG. 20, for example).

The tapered concave surface 121 is configured to produce a substantially convergent jet 135 of fluid mist, as shown in **FIGS. 14A, B,** for example. **FIG. 14A** is a side view of an ultrasonic blade comprising a tapered concave blade tip depicting the convergent jet 135 of fluid mist emanating from the distal end of the blade 120 in direction A. **FIG. 14B** is a detail view of the convergent jet 135 of fluid mist. The convergent jet 135 may be produced by the tapered concave shape of distal end 134 of the blade body 122. Fluid droplets 139 that collide with the tapered concave shape of the distal end 134 of the blade body 122 will tend to converge rather than diverge as the fluid droplets 139 travel away from the distal end 134 of the blade body 122 in the direction of arrow A. Generally, when the fluid droplets 139 collide with a convex shaped blade tip, the fluid particles 139 tend to produce a substantially divergent jet of fluid mist 137, as shown in **FIG. 13A, B,** for example. **FIG. 13A** is a side view of an ultrasonic blade 820 with a convex blade tip depicting a typical divergent jet 137 of fluid mist. **FIG. 13B** is a detail view of the divergent jet 137 of fluid mist. For example, when fluid particles associated with the surgical site collide with a convex shaped distal end of a blade body, the fluid mist that emanates from the distal end 134 of the blade body in direction A, tends to produce the divergent jet 137 of fluid mist, as shown in FIG. 13A. This fluid mist may limit the visibility at the surgical site.

As shown in FIG. 14B, the tapered concave surface 121 may cause the fluid droplets moving in direction A to be directed towards the longitudinal axis 127 where the fluid droplets 141 may collide and coalesce, thus increasing droplet size such that the fluid droplets 141 may drop out under the influence of gravity 142.

With reference now back to FIGS. 4-6, in various embodiments, the distal end 134 may comprise a first edge 124. The first edge 124 may form the base from which the tapered surface 121 extends inwardly into the blade body 122 in the direction B. The first edge 124 may be formed in a variety of shapes including a circle, an ellipse, a square, a rectangle, a pentagon, a hexagon or any suitable polygon. In one embodiment, as shown in FIGS. 4-6, the tapered concave surface 121 defines a conical shape extending inwardly in direction B into the blade body 122. The conical shape may comprise a cone with an apex 126 and a circular base. In other embodiments, the base may be an ellipse, or a polygon (e.g., a pyramid) and may also comprise a right cone (e.g., where a line joining the apex to the center of the base is at a right angle to the base plane) or an oblique cone (e.g., where a line joining the apex to the center of the base is not at a right angle to the base plane). The surface may terminate at the apex 126 within the blade body 122. The conical shape of the tapered concave surface 121 may be symmetrical or asymmetrical. In the embodiment illustrated in FIGS. 4-6, the conical shape is symmetric with the apex located substantially along the longitudinal axis 127. In other embodiments, the conical shape of the tapered concave surface 121 may be asymmetric with the apex 126 located between an outer edge 159 of the blade body 122 and the longitudinal axis 127.

In various other embodiments, the tapered concave surface 221 of the blade body 122 may define various other symmetrical or asymmetrical shapes. In one embodiment, as shown in **FIGS. 7-9,** the tapered concave surface 221 may define a frusto-conical shape. **FIG. 7** is a side view of another embodiment of the ultrasonic blade 220. **FIG. 8** is a cross-sectional view of the ultrasonic blade 220 taken along line 8-8 in FIG. 7. **FIG. 9** is a perspective view of the ultrasonic blade 220 in FIG. 7. The frusto-conical shape may extend inwardly into the blade body 122 in direction B from the first edge 124. The frusto-conical shape may comprise all of the characteristics of a cone, as defined above, but may terminate short of a hypothetical apex of the cone, in other words, the frusto-conical shape may be a shape similar to a cone but terminating in a plane 227 substantially orthogonal to the longitudinal axis 127 as opposed to a point along or near the longitudinal axis 127 found in a cone. The tapered concave surface 221 may terminate prior to reaching the hypothetical apex within the blade body 122. For example, the frusto-conical shape may be a cone with a substantially flat top as opposed to a point. In various other embodiments, the frusto-conical shape may have a rounded top or any other suitable shape for the top portion. In the embodiments illustrated in FIGS. 7-9, the frusto-conical shape of the tapered concave surface 221 is symmetric with the center 131 of the plane 227 located substantially along the longitudinal axis 127. In other embodiments, the frusto-conical shape of the tapered concave surface 221 may be asymmetric with the center 131 of the plane 227 located between an outer edge 129 of the blade body 122 and the longitudinal axis 127.

In another embodiment, as shown in **FIGS. 10-12,** the ultrasonic blade 320 comprises a tapered concave surface 321 defining a partial spheroid extending inwardly into the blade body 122 in the direction B. **FIG. 10** is a side view of the ultrasonic blade 320. **FIG. 11** is a cross-sectional view of the ultrasonic blade 320 taken along line 11-11 in FIG. 10. **FIG. 12** is a perspective view of the ultrasonic blade 320 in FIG. 10. The partial spheroid may extend inwardly from the first edge 124, or base, into the blade body 122 in the direction of B. A spheroid may be formed when an ellipse or circle is rotated about an axis. For example, when a circle is rotated about its axis, a spheroid, commonly referred to in this case as a sphere, is formed. When the ellipse is rotated about its major axis a prolate spheroid is formed, and when the ellipse is rotated about its minor axis an oblate spheroid is formed. The tapered concave surface 321 may define at least one of a partial sphere, a partial prolate spheroid, or a partial oblate spheroid. The partial spheroid may be more than half of a spheroid, less than half of a spheroid, or exactly half of a spheroid (e.g., a hemispheroid). The first edge 124 may form a circle or an ellipse which has a center 133 that may be substantially aligned with the longitudinal axis 127.

In at least one embodiment, the blade may comprise a variety of shapes. For example, the blade may be curved. The blade may be curved in any direction. In addition, the blade may comprise various cross-sections. For example, the blade may comprise a square cross-section. All of these blade shapes may comprise an axis defined between the proximal end 132 and the distal end 134 of the blade.

**FIG. 23** is a perspective view of an asymmetric ultrasonic blade comprising a tapered concave surface extending inwardly into the blade body. More details regarding curved or asymmetric blades are described in U.S. Patent No. 6,283,981. As shown in FIG. 23, the ultrasonic surgical instrument 10 may comprise an ultrasonic blade 920 and a treatment region 960 that includes a curved blade designed to cut and coagulate tissue. The treatment region 960 may be curved to provide the surgeon with better access and visibility. The treatment region 960 may also comprise a tapered concave surface 921 which may provide a mist reducing feature. As illustrated in FIG. 23, the curved treatment region may be symmetrical about x,z plane, but asymmetrical about x,y plane. The tapered concave surface 921 may extend inwardly into the blade body 922 from a first edge 924 which may extend substantially parallel to the perimeter of the blade tip 923. In other embodiments, the first edge may be a different shape from the perimeter of the blade tip. For example, the first edge may form a circle when the perimeter of the blade tip forms a trapezoid. The embodiments are not limited in this context.

As previously discussed, in various embodiments, the tapered concave surface may extend inwardly into the blade body 122 in direction B from a first edge 124 either symmetrically or asymmetrically. This extension may occur at or near the longitudinal central axis 127 of the blade body 122. For example, with respect to the embodiment illustrated in FIGS. 4-6, the surface may extend symmetrically to form or define a right cone or asymmetrically to form or define an oblique cone. **FIG. 21** is a side view of an ultrasonic blade 820 with a tapered concave surface 821 extending inwardly into the blade body 122 asymmetrically along direction B. **FIG. 22** is a cross-sectional view of the ultrasonic blade 820 taken along line 22-22 in FIG. 21. As shown in FIG. 21, the tapered concave surface 821 extends inwardly from the distal end 134 of the blade 820 to the proximal end 132 of the blade 820 to form a substantially oblique cone. The oblique cone may be formed asymmetrically about the longitudinal axis 127. For example, the apex 826 of the oblique cone may be offset from the center of the longitudinal axis 127 or the center 143 of the geometric shape formed by the first edge 124. The surface may form any geometrical shape, which may be formed asymmetrically within the blade body.

In various embodiments, as shown in **FIGS. 15A-D,** at least a portion 129 of the blade body 122 may comprise a layer of material 150 to minimize the divergent jet 137 of fluid mist (FIGS. 13A, B) associated with the ultrasonic blade 420. **FIG. 15A** is a side view of an ultrasonic blade 420 with at least a portion 129 of the ultrasonic blade 420 comprising at least one layer of the material 150 formed thereon. **FIG. 15B** is cross-sectional view of the ultrasonic blade 420 taken along line 15B-15B in FIG. 15A. **FIG. 15C** is a detailed view of the ultrasonic blade 420 of FIG. 15A. The coated portion 129 of the blade body 122 may be located at the distal end 134 of the ultrasonic blade 420. The coated portion 129 of the blade body 122 may comprise at least one layer of a material 150 which acts to globulize fluid particles 152 when they contact the coated portion 129 of the blade body 122. To globulize refers to creating globules or forming droplets of fluid. The material 150 may have properties which cause the material 150 to repel fluid. For example, the material 150 may be hydrophobic and thus repel fluid which may include irrigation saline, interstitial fluid, blood plasma and a cell.

The gobulization of the fluid may be caused by differences between the surface tension of the material 150 and the surface tension of the fluid in contact with the material 150. The material 150 may have a surface tension which is less than the surface tension of the fluid which may cause the fluid to globulize on the surface of the material 150. A fluid may form globules or "beads" on surfaces coated with a material where the surface tension of the material 150 on the surface 156 is less than the surface tension of the fluid. The formation of globules may prevent the "wetting" or formation of a layer of fluid spreading over the surface of the coated portion 129 of the blade body 122. The globules 152 may be pushed off of the blade body 122 through the vibrating motion of the end effector 50 unlike a layer of fluid which may have to be atomized from the surface thus causing a mist to form. The effects of the differences between the surface tension of the material 150 and the surface tension of the fluid may be illustrated in terms of a contact angle formed between a fluid interface and a surface.

**FIG. 15D** illustrates a contact angle 156 formed between a fluid interface 157 and a surface 158 of the ultrasonic blade 122 of FIG. 15A. As shown in FIG. 15D, the contact angle 156 is the angle at which the fluid interface 157 meets the surface 158 of the material 150. The contact angle 156 is specific for any given system and is determined by the interactions across the three interfaces. For clarity, the concept is illustrated with a small liquid droplet resting on a flat horizontal solid surface. On extremely hydrophilic surfaces, a water droplet will completely spread (an effective contact angle of 0°). This occurs for surfaces that have a large affinity for water (including materials that absorb water). On many hydrophilic surfaces, water droplets will exhibit contact angles of 10° to 30°, for example. On highly hydrophobic surfaces, which are incompatible with water, one may observe a large contact angle (70° to 90°). Some surfaces have water contact angles as high as 150° or even nearly 180°. On these surfaces, water droplets simply rest on the surface, without actually wetting the surface to any significant extent, for example. These surfaces are termed superhydrophobic and can be obtained on fluorinated surfaces (TEFLON®-like coatings) that have been appropriately micropatterned. The contact angle 156 thus directly provides information on the interaction energy between the surface 156 of the material 150 and the fluid.

In various embodiments, the surface 158 of the material 150 may be hydrophobic or superhydrophobic. The first material 150 may comprise any one of polytetrafluoroethylene (TEFLON®), polypropylene, polyethylene, waxes, polycaprolactone, any combination thereof, or any other suitable hydrophobic or superhydrophobic material. For example, the first material 150 may comprise at least one of a polypropylene wax hydrocarbon mixture or TEFLON®. The first material 150 may be applied to the surface through a variety of coating techniques including dipping, spraying, brushing, drying, melting, sintering, fused curing, and any other suitable method for applying hydrophobic materials. Other methods for applying hydrophobic materials may include material deposition techniques that are well known in the art. More details regarding hydrophobic and superhydrophobic materials and methods for applying those materials to a surface are described by U.S. Patent No. 7,041,088 and U.S. Patent No. 6,663,941.

In various other embodiments, as shown in **FIGS. 16-17,** at least a portion of the blade body 122 may be coated with at least two materials which may allow an electric charge to be carried by at least one of the materials. **FIG. 16** is a side view of an ultrasonic blade 520 with portions of the blade body 122 coated with more than one material to provide an electric charge to the distal end 134 of the blade body 122. **FIG. 17** is cross-sectional view of the ultrasonic blade 520 taken along line 17-17 in FIG. 16. At least a first portion 129 of the blade body 122 may comprise at least one layer of a first material 160. This first material 160 may contact at least a portion of a second material 162. The first material 160 may comprise a material suitable to carry an electric charge. The electric charge carried by the first material 160 may be the same as the nominal electric charge carried by the fluid. The similar electric charges may cause the portion 129 of the blade body 122 covered with the first material to repel the fluid. For example, if the first material 160 has a positive charge and the fluid has a positive charge, the fluid will be repelled by the first material 160. Accordingly, the first material 160 acts as a hydrophobic surface. The first material 160 may receive its electrical charge carried by wires from an electrical source located at or near the proximal end 132 of the blade body 122. For example, the electrical source may comprise a direct current ("DC") electrical source (e.g., a battery). In another embodiment, the electrical source may be located in a different location. The wires may be provided within a bore formed in the ultrasonic blade 520 or maybe provided along the outside of the ultrasonic blade 520 within a channel or conduit. The misting effect may be reduced because the fluid is repelled from the surface of the first material 160. Accordingly, there is minimal fluid on the surface of the blade body 122 to be atomized by the ultrasonically activated blade 520.

At least a second portion of the blade body 122 comprises at least one layer of a second material 162. The second material 162 may comprise an electrically insulative material. The second material 162 may be located between the first material 160 and the blade body 122. The second material 162 may insulate the blade 520, and the blade body 122, from electrical charges. The second material 162 may be an electret material which may be made from silicon dioxide, fluoropolymer, polypropylene or any other suitable material. These materials may hold a constant or slow decaying charge. The first material 160 may be a metallic layer or a vapor deposited layer acting as a floating conductor wherein wires may not be required to convey a charge to the second material 162 from an electrical source.

In another embodiment, the electric charge carried by the first material 160 may be the opposite polarity as the nominal electric charge carried by the fluid. The opposite electric charges may cause the portion 129 of the blade body 122 covered with the first material to attract the fluid. For example, if the first material 160 has a negative charge and the fluid has a positive charge, the fluid will be attracted by the first material 160. Accordingly, the first material 160 acts as a hydrophilic surface. Accordingly, electric charge on the coating materials may be selected such that they exhibit opposite charges to that of the fluid to create attraction rather than repulsion between the blade body 122 and the fluid. This may enable surgical "smoke" or mist to globulize as it collects on the surface of the blade body 122. In addition, this technique may be employed to attract other materials or constituents, such as, drug molecules, fibrin, and natural adhesives to the treatment site. These other materials or constituents may be introduced in a liquid suspension. The difference in charges between the blade body 12 ad the fluid would act to concentrate these other materials or constituents in the vicinity of the distal end of the blade body 122.

In various embodiments, as shown in **FIGS. 18-19,** a blade 620 may comprise a bore 180 (e.g., a lumen). **FIG. 18** is a side view of the ultrasonic blade 620 with a longitudinally extending bore 180. **FIG. 19** is cross-sectional view of the ultrasonic blade 620 taken along line 19-19 in FIG. 18. The bore 180 may extend longitudinally along the longitudinal axis 127, or, in certain embodiments, the bore may extend in a different direction. The bore 180 may be formed within the blade 620. The ultrasonic blade 620 may be configured to emit a spray via the bore 180 in a direction indicated by arrow 640 at the distal end 134 of the blade 620. The spray may emanate from a spray source 161 located at or near the proximal end 132 of the blade 620 and travel in the flow direction 640. The flow direction 640 may be from the proximal end 132 to the distal end of the blade 620. In another embodiment, the spray source 161 may be found in other locations. The spray emanating from the distal end 134 of the blade 620 may substantially prevent fluid from contacting the distal end 134 of the blade 620. This prevention of contact may reduce the mist as a layer of fluid may not be present on the blade 620 for atomization. The spray may comprise a gas. For example, the gas may be carbon dioxide, air or some other suitable gas.

The ultrasonic blade 120 comprises a treatment region 128 that is suitable to effect tissue, such as, for example, cut, coagulate, reshape, scrape, and remove tissue. A distal end 134 of the treatment region 128 may also comprise a tip with a cutting edge. Additional cutting edges may be positioned laterally along both sides of the treatment region 128. In one embodiment, the cutting edges extend from the proximal end 132 to the distal end 134 of the treatment region 128.

The ultrasonic blades as discussed herein may be fabricated from a material suitable for transmission of ultrasonic energy such as, for example, Ti6A14V, Aluminum, Stainless Steel, or other known materials. The ultrasonic blade may be used in a single-element end effector (e.g., a scalpel, hook, or ball coagulator) as discussed with reference to ultrasonic system 10 and FIGS. 1A, 2 and 3A, or a multiple-element end effector (e.g., clamping coagulating shears) as discussed with reference to ultrasonic system 1000 and FIGS. 1B, 3B, and 3C, for example.

The devices disclosed herein can be designed to be disposed of after a single use, or they can be designed to be used multiple times. In either case, however, the device can be reconditioned for reuse after at least one use. Reconditioning can include any combination of the steps of disassembly of the device, followed by cleaning or replacement of particular pieces, and subsequent reassembly. In particular, the device can be disassembled, and any number of the particular pieces or parts of the device can be selectively replaced or removed in any combination. Upon cleaning and/or replacement of particular parts, the device can be reassembled for subsequent use either at a reconditioning facility, or by a surgical team immediately prior to a surgical procedure. Those skilled in the art will appreciate that reconditioning of a device can utilize a variety of techniques for disassembly, cleaning/replacement, and reassembly. Use of such techniques, and the resulting reconditioned device, are all within the scope of the present application.

Preferably, the various embodiments described herein will be processed before surgery. First, a new or used instrument is obtained and if necessary cleaned. The instrument can then be sterilized. In one sterilization technique, the instrument is placed in a closed and sealed container, such as a plastic or TYVEK® bag. The container and instrument are then placed in a field of radiation that can penetrate the container, such as gamma radiation, x-rays, or high-energy electrons. The radiation kills bacteria on the instrument and in the container. The sterilized instrument can then be stored in the sterile container. The sealed container keeps the instrument sterile until it is opened in the medical facility.

It is preferred that the device is sterilized. This can be done by any number of ways known to those skilled in the art including beta or gamma radiation, ethylene oxide, steam.

Although various embodiments have been described herein, many modifications and variations to those embodiments may be implemented. For example, different types of end effectors may be employed. In addition, combinations of the described embodiments may be used. For example, a concave blade tip may be coated with a hydrophobic material. Also, where materials are disclosed for certain components, other materials may be used. The foregoing description and following claims are intended to cover all such modification and variations.

Any patent, publication, or other disclosure material, in whole or in part, that is mentioned should be considered only to the extent that the disclosed material does not conflict with existing definitions, statements, or other disclosure material set forth in this disclosure. As such, and to the extent necessary, the disclosure as explicitly set forth herein supersedes any mentioned conflicting material.

**Claims**

1. An ultrasonic surgical blade (50) coupled to an ultrasonic transmission waveguide (104) fabricated from a solid core shaft, the surgical blade comprising:

   a body (122) defining a longitudinal axis, the body having a proximal end and a distal end, wherein the distal end is movable relative to the longitudinal axis by the vibrations produced by a transducer;
   a treatment region (128) extending from the proximal end to the distal end; and
   a tapered concave surface (121) formed at the distal end extending inwardly into the blade body,
   **characterized in that** the tapered concave surface (121) is configured to produce a substantially convergent jet (135) of fluid mist directed towards the longitudinal axis (127).

2. The ultrasonic surgical blade and ultrasonic transmission waveguide of claim 1, wherein the ultrasonic blade further comprises:

   a first edge (124); and
   a substantially circular cross-section.

3. The ultrasonic surgical blade and ultrasonic transmission waveguide of claim 2, wherein the first edge forms a base of the tapered concave surface.

4. The ultrasonic surgical blade and ultrasonic transmission waveguide of any preceding claim, wherein the treatment region comprises at least one coagulating edge.

5. The ultrasonic surgical blade and ultrasonic transmission waveguide of any one of claims 1-4, wherein the tapered concave surface is an asymmetric surface.

6. The ultrasonic surgical blade and ultrasonic transmission waveguide of any one of claims 1-4, wherein the tapered concave surface is a substantially concave surface that comprises a convex portion.

7. The ultrasonic surgical blade and ultrasonic transmission waveguide of any one of claims 1-4, wherein the tapered concave surface is a surface that defines a conical shape extending inwardly into the blade body and wherein the tapered concave surface terminates at an apex (126) within the blade body.

8. The ultrasonic surgical blade and ultrasonic transmission waveguide of any one of claims 1-4, wherein the tapered concave surface is a surface that defines a frusto-conical shape extending inwardly into the blade body and wherein the tapered concave surface terminates prior to reaching the apex (126) within the blade body.

9. The ultrasonic surgical blade and ultrasonic transmission waveguide of any one of claims 1-4, wherein the tapered concave surface is a surface that defines a partial spheroid extending inwardly into the blade body.

10. The ultrasonic surgical blade and ultrasonic transmission waveguide of any preceding claim, wherein the treatment region comprises at least one cutting edge.

11. A surgical instrument (10), comprising:

    a transducer (14) configured to produce vibrations at a predetermined frequency;
    the ultrasonic blade (50) and ultrasonic transmission waveguide (104) of any preceding claim extending along the longitudinal axis and coupled to the transducer.

**Patentansprüche**

1. Ultraschalloperationsmesser (50), das mit einem Ultraschallübertragungswellenleiter (104) gekoppelt ist, der aus einer Vollkernwelle hergestellt wurde, wobei das Operationsmesser umfasst:

   einen Körper (122), der eine Längsachse definiert, wobei der Körper ein proximales Ende und ein distales Ende aufweist, wobei das distale Ende bezogen auf die Längsachse durch die von einem Wandler erzeugten Vibrationen bewegt werden kann;
   einen Behandlungsbereich (128), der sich von dem proximalen Ende bis zu dem distalen Ende erstreckt; und
   eine konische konkave Oberfläche (121), die an dem distalen Ende gebildet ist und sich nach innen in den Messerkörper erstreckt,
   **dadurch gekennzeichnet, dass** die konische konkave Oberfläche (121) dazu ausgelegt ist, einen im Wesentlichen konvergenten Strahl (135) aus Fluidnebel zu erzeugen, der auf die Längsachse (127) gerichtet wird.

2. Ultraschalloperationsmesser und Ultraschallübertragungswellenleiter nach Anspruch 1, wobei das Ultraschallmesser ferner umfasst:

   eine erste Kante (124); und
   einen im Wesentlichen kreisförmigen Querschnitt.

**3.** Ultraschalloperationsmesser und Ultraschallübertragungswellenleiter nach Anspruch 2, wobei die erste Kante eine Basis der konischen konkaven Oberfläche bildet.

**4.** Ultraschalloperationsmesser und Ultraschallübertragungswellenleiter nach einem der vorhergehenden Ansprüche, wobei der Behandlungsbereich mindestens eine koagulierende Kante umfasst.

**5.** Ultraschalloperationsmesser und Ultraschallübertragungswellenleiter nach einem der Ansprüche 1-4, wobei die konische konkave Oberfläche eine asymmetrische Oberfläche ist.

**6.** Ultraschalloperationsmesser und Ultraschallübertragungswellenleiter nach einem der Ansprüche 1-4, wobei die konische konkave Oberfläche eine im Wesentlichen konkave Oberfläche ist, die einen konvexen Abschnitt umfasst.

**7.** Ultraschalloperationsmesser und Ultraschallübertragungswellenleiter nach einem der Ansprüche 1-4, wobei die konische konkave Oberfläche eine Oberfläche ist, die eine konische Form definiert, die sich nach innen in den Messerkörper erstreckt, und wobei die konische konkave Oberfläche an einer Spitze (126) im Innern des Messerkörpers endet.

**8.** Ultraschalloperationsmesser und Ultraschallübertragungswellenleiter nach einem der Ansprüche 1-4, wobei die konische konkave Oberfläche eine Oberfläche ist, die eine kegelstumpfförmige Form definiert, die sich nach innen in den Messerkörper erstreckt, und wobei die konische konkave Oberfläche vor dem Erreichen der Spitze (126) im Innern des Messerkörpers endet.

**9.** Ultraschalloperationsmesser und Ultraschallübertragungswellenleiter nach einem der Ansprüche 1-4, wobei die konische konkave Oberfläche eine Oberfläche ist, die ein partielles Sphäroid definiert, das sich nach innen in den Messerkörper erstreckt.

**10.** Ultraschalloperationsmesser und Ultraschallübertragungswellenleiter nach einem der vorhergehenden Ansprüche, wobei der Behandlungsbereich mindestens eine Schnittkante umfasst.

**11.** Operationsinstrument (10), das umfasst:

einen Wandler (14), der dazu ausgelegt ist, Vibrationen mit einer vorbestimmten Frequenz zu erzeugen;
das Ultraschallmesser (50) und den Ultraschallübertragungswellenleiter (104) nach einem der vorhergehenden Ansprüche, die sich entlang der Längsachse erstrecken und mit dem Wandler gekoppelt sind.

**Revendications**

**1.** Lame chirurgicale à ultrasons (50) couplée à un guide d'ondes de transmission d'ultrasons (104) fabriqué à partir d'un arbre à noyau massif, la lame chirurgicale comprenant :

un corps (122) définissant un axe longitudinal, le corps ayant une extrémité proximale et une extrémité distale, dans laquelle l'extrémité distale est mobile par rapport à l'axe longitudinal du fait des vibrations produites par un transducteur ;
une région de traitement (128) s'étendant de l'extrémité proximale à l'extrémité distale ; et
une surface concave effilée (121) réalisée à l'extrémité distale s'étendant vers l'intérieur dans le corps de la lame,
**caractérisée en ce que** la surface concave effilée (121) est configurée pour produire un jet sensiblement convergent (135) de brouillard de fluide dirigé vers l'axe longitudinal (127).

**2.** Lame chirurgicale à ultrasons et guide d'ondes de transmission d'ultrasons selon la revendication 1, dans laquelle la lame à ultrasons comprend en outre :

un premier bord (124) ; et
une section transversale sensiblement circulaire.

**3.** Lame chirurgicale à ultrasons et guide d'ondes de transmission d'ultrasons selon la revendication 2, dans laquelle le premier bord forme une base de la surface concave effilée.

**4.** Lame chirurgicale à ultrasons et guide d'ondes de transmission d'ultrasons selon l'une quelconque des revendications précédentes, dans laquelle la région de traitement comprend au moins un bord coagulant.

**5.** Lame chirurgicale à ultrasons et guide d'ondes de transmission d'ultrasons selon l'une quelconque des revendications 1 à 4, dans laquelle la surface concave effilée est une surface asymétrique.

**6.** Lame chirurgicale à ultrasons et guide d'ondes de transmission d'ultrasons selon l'une quelconque des revendications 1 à 4, dans laquelle la surface concave effilée est une surface sensiblement concave qui comprend une partie convexe.

**7.** Lame chirurgicale à ultrasons et guide d'ondes de transmission d'ultrasons selon l'une quelconque des revendications 1 à 4, dans laquelle la surface concave effilée est une surface qui définit une forme conique s'étendant vers l'intérieur dans le corps de la lame et dans laquelle la surface concave effilée se termine à un sommet (126) au sein du corps de la lame.

8. Lame chirurgicale à ultrasons et guide d'ondes de transmission d'ultrasons selon l'une quelconque des revendications 1 à 4, dans laquelle la surface concave effilée est une surface qui définit une forme tronconique s'étendant vers l'intérieur dans le corps de la lame et dans laquelle la surface concave effilée se termine avant d'atteindre le sommet (126) au sein du corps de la lame.

9. Lame chirurgicale à ultrasons et guide d'ondes de transmission d'ultrasons selon l'une quelconque des revendications 1 à 4, dans laquelle la surface concave effilée est une surface qui définit un sphéroïde partiel s'étendant vers l'intérieur dans le corps de la lame.

10. Lame chirurgicale à ultrasons et guide d'ondes de transmission d'ultrasons selon l'une quelconque des revendications précédentes, dans laquelle la région de traitement comprend au moins un bord tranchant.

11. Instrument chirurgical (10), comprenant:

un transducteur (14) configuré pour produire des vibrations à une fréquence prédéterminée ; la lame à ultrasons (50) et le guide d'ondes de transmission d'ultrasons (104) de l'une quelconque des revendications précédentes s'étendant le long de l'axe longitudinal et étant couplés au transducteur.

FIG.1A

FIG. 1B

FIG. 2

FIG. 3A

FIG. 3C

FIG. 3B

FIG. 4

FIG. 5

FIG. 6

FIG. 7

FIG. 8

FIG. 9

FIG. 10

FIG. 11

FIG. 12

FIG. 13A

EP 2 178 448 B1

FIG. 13B

FIG. 14A

FIG. 14B

EP 2 178 448 B1

420

15B

152  152  152    129   128        159        132        130

150

134          122         127

152

152  152

122

15B

A        B

FIG. 15A

150

159        122

FIG. 15B

FIG. 15C

FIG. 15D

FIG. 16

FIG. 17

EP 2 178 448 B1

FIG.18

SPRAY SOURCE

FIG.19

FIG. 20

EP 2 178 448 B1

**FIG. 21**

**FIG. 22**

FIG. 23

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 11881643 B
- US 5897569 A
- US 6425906 B1
- EP 0238667 A1
- US 5322055 A
- US 5954736 A
- US 6309400 B2
- US 6278218 B1
- US 6283981 B1
- US 6325811 B1
- US 6283981 B
- US 7041088 B
- US 6663941 B